# EUROPÄISCHE PATENTSCHRIFT

(11) **EP 0 598 867 B1**
(45) Veröffentlichungstag und Bekanntmachung des Hinweises auf die Patenterteilung: **05.03.1997**
(21) Anmeldenummer: 93909760.6
(22) Anmeldetag: 10.06.1993
(51) Int. Cl.: F16N 11/10, A61M 5/155

(54) **VORRICHTUNG ZUR GEZIELTEN, STEUERBAREN ABGABE EINER FLÜSSIGKEIT ODER EINER VISKOSEN MASSE**
DEVICE FOR THE INTENTIONAL AND CONTROLLABLE DISTRIBUTION OF A LIQUID OR VISQUOUS MATERIAL
DISPOSITIF PERMETTANT DE DISTRIBUER UN LIQUIDE OU UNE MATIERE VISQUEUSE DE MANIERE CIBLEE ET CONTROLEE

(30) Priorität: 15.06.1992 CH 1870/92
(43) Veröffentlichungstag der Anmeldung: 01.06.1994
(73) Patentinhaber: WYSSMANN, Max, CH-3360 Herzogenbuchsee (CH)
(72) Erfinder: WYSSMANN, Max, CH-3360 Herzogenbuchsee (CH)
(74) Vertreter: Specht, Peter, Dipl.-Phys.
(86) Internationale Anmeldenummer: CH9300151
(87) Internationale Veröffentlichungsnummer: WO9325841

(56) Entgegenhaltungen:
- EP-A- 0 278 138
- EP-A- 0 385 916
- WO-A-88/04751
- WO-A-88/09187
- WO-A-88/09901
- WO-A-89/08800
- US-A- 4 414 298
- PATENT ABSTRACTS OF JAPAN vol. 13, no. 295 (E-783)7. Juli 1989 & JP-A-10 76 665 (SHIMADA KENJI) 22 March 1989

## Beschreibung

Die Erfindung betrifft eine Vorrichtung nach dem Oberbegriff des Anspruches 1. Eine derartige Vorrichtung ist aus der WO-8 908 800 bekannt.

### Technisches Gebiet

Förderung kleiner Mengen vergleichsweise wertvoller flüssiger oder viskoser Substanzen nach vorher festgelegtem, genau einzuhaltendem Zeitplan. Selbsttätige, autonome Steuerung des Programmablaufs im wesentlichen ohne Eingriff von aussen.

Die Erfindung bezieht sich auf die Weiterentwicklung, Vervollkommnung und Erweiterung des Verwendungsbereichs von mit Gasentwicklungszellen als Primärenergieträger angetriebenen Fördermechanismen für leicht- bis zähflüssige Medien und verwandte Fördergüter.

Im engeren Sinne betrifft die Erfindung eine Vorrichtung zur gezielten, steuerbaren Abgabe einer Flüssigkeit oder einer viskosen Masse oder einer Suspension von Feststoffpartikeln in einer Flüssigkeit, bestehend aus einem zylindrischen Behälter, einem Behälterabschluss mit Schraub- oder Steckanschluss, einem in den Behälter mit Schiebesitz passenden, letzteren in einen Druckgasraum und einen Masseraum unterteilenden zylindrischen Kolben mit ringförmiger Dichtung am Umfang, einem die elektrischen Elemente wie elektrochemische Gasentwicklungszellen, Einstell- und Belastungswiderstände und Kontakte enthaltenden Bauteil.

### Stand der Technik

Vorrichtungen zur Förderung von flüssigen oder halbflüssigen, viskosen,fetten oder teigigen Massen unter Herbeiziehung eines von einer elektrochemischen Gasentwicklungszelle bereitgestellten Druckgases als kraftausübendes Medium sind bereits bekannt. Ebenfalls bekannt ist die Gasentwicklungszelle selbst, deren Prinzip auf elektrochemischen Umsetzungen in einem Primärelement beruht. Dabei wird aus dem Elektrolyt ein Gas, meist Wasserstoff freigesetzt. Da bei dieser Art elektrochemischer Zelle die Gasentwicklung unter der Voraussetzung der Konstanthaltung der übrigen Betriebsparameter nur vom internen Strom abhängig ist, liefert sie eine diesem proportionale Gasmenge pro Zeiteinheit. Der Strom ist vom Gesamtwiderstand des geschlossenen Stromkreises abhängig und somit durch den äusseren Widerstand einstellbar bzw. steuer- und regelbar. Dadurch ergibt sich eine Einstellbarkeit bzw. Steuerbarkeit der Gasproduktion pro Zeiteinheit.

Von den zahlreichen Typen angebotener Gasentwicklungszellen interessiert hier praktisch nur diejenige in Kompaktbauweise mit Gasdiffusionselektrode. Die Zelle wird im allgemeinen durch Schliessen des äusseren Stromkreises in Betrieb gesetzt.

Es sind bereits Vorrichtungen zur Förderung von Schmiermitteln bekannt, welche sich einer Membran oder eines Kolbens als druckübertragendes Bauelement zum Ausstossen des Schmierfettes in Funktion der Zeit bedienen. Es sind schon verschiedene Konstruktionen vorgeschlagen worden, bei denen zum Beispiel das erzeugte Gas einen ausdehnbaren Körper erweitert, der seinerseits auf eine Membran oder einen Kolben wirkt, oder wobei eine Zelle direkt in den Behälterdeckel oder in den Kolben auf der Druckgasseite der Vorrichtung eingebaut ist. In anderen Fällen wurde das Schmiermittel in zunächst geschlossenem Beutel in den Zylinder der Vorrichtung eingeführt und der Beutel als Ganzes dem Druck eines Kolbens ausgesetzt. Bei allen bekannten Kolbenanordnungen wurden zur betriebssicheren Abtrennung des Druckgasraumes vom Masseraum spezielle, meist ringförmige Dichtungselemente verwendet, eine Dichtungsart, deren Prinzip vom allgemeinen Kolbenmaschinenbau her bekannt ist. Der Behälter ist meistens mit einem zwecks Nachfüllung des Schmiermittels aufschraubbaren oder aufsteckbaren trichterförmigen Behälterabschluss versehen.

Es sind ferner Vorrichtungen bekannt, die zur Förderung von pharmazeutischen oder medizinischen Mitteln in Form von Infusionslösungen für Patienten dienen. Dabei werden meistens die zylindrischen Gefässe von Ampullen oder Infusionsspritzen als Behälter verwendet, in denen sich ein durch Gasdruck getriebener Kolben bewegt. An der offenen Stirnseite des Behälters befindet sich ein die gaserzeugenden und die elektrischen Steuerelemente enthaltender, den Behälter gasdicht abschliessender Einsatz. Am Behälterausgang befindet sich eine aufsteckbare Injektionsnadel ein Katheter oder eine Drosseleinrichtung.

Die bekannten Vorrichtungen zur Förderung von Massen mittels Gasentwicklungszellen als Primärenergieträger lassen in konstruktiver wie in betrieblicher Hinsicht sehr zu wünschen übrig. Dies bezieht sich insbesondere auf Details und Anzahl der konstituierenden Bauelemente, auf die Wartung, die Lebensdauer, die Nachfüllbarkeit, die Wiederverwendbarkeit besonders der wertvolleren Bestandteile und der Robustheit und Betriebssicherheit. Die bekannten Ausführungen sind in ihrem Aufbau oft zu kompliziert, zu unhandlich und zu stark dem Verschleiss unterworfen.

Es besteht daher ein grosses Bedürfnis zur Weiterentwicklung, Verbesserung, Vervollkommnung und Vereinfachung derartiger Vorrichtungen im allgemeinen wie im speziellen (Schmierstoffgeber, Infusionsgeräte etc.).

Zum Stand der Technik werden auch noch folgende Druckschriften zitiert:
- DE-C-35 32 335
- EP-B-0 278 954
- DE-A-37 18 341
- WO-88/09187

### Darstellung der Erfindung

Der Erfindung liegt die Aufgabe zugrunde, eine Vorrichtung zur gezielten steuerbaren Abgabe einer Flüssigkeit, einer viskosen Masse oder einer Suspension von Feststoffpartikeln in einer Flüssigkeit anzugeben, wobei von der Druckausübung eines von einer elektrochemischen Gasentwicklungszelle stammenden Gases Gebrauch gemacht wird. Die Vorrichtung soll bei möglichst weitreichender Verwendungsmöglichkeit und Universalität als Ganzes eine lange Lebensdauer, geringen Verschleiss und einfache kostensparende Wartung aufweisen, in ihrem konstruktiven Aufbau möglichst einfach sein und mit einer minimalen Anzahl von einzelnen Bauteilen auskommen. Die Auswechslung von dem Verbrauch oder Verschleiss unterworfenen Elementen und das Nachfüllen der zu fördernden Masse soll ohne Verschmutzung, ohne Korrosionsangriff oder Beeintächtigung des guten elektrischen Kontaktes erfolgen. Auf spezielle separate Dichtungselemente sollte verzichtet werden. Visuelle und akustische Anzeige- und Warneinrichtungen sollen einfacher und betriebssicherer Art sein. Schliesslich soll die Vorrichtung möglichst kostengünstig ausfallen und sich für rationelle Massenfabrikation eignen.

Diese Aufgabe wird durch den Gegenstand des Anspruches 1 gelöst.

### Weg zur Ausführung der Erfindung

Die Erfindung wird anhand der nachfolgenden, durch Figuren näher erläuterten Ausführungsbeispiele beschrieben.

Dabei zeigt:
- Fig. 1: eine schematische Darstellung in Längsschnitt (Aufriss) des prinzipiellen Aufbaus der Vorrichtung, gleichzeitig Darstellung einer Ausführungsform eines Schmierstoffgebers,
- Fig. 2: eine Darstellung des Grundrisses des äusseren steckbaren stopfenförmigen Bauteils zur Einstellung des Belastungswiderstandes eines Schmierstoffgebers,
- Fig. 3: eine Darstellung des Grundrisses des Behälters, Behälterbodenseite, eines Schmierstoffgebers,
- Fig. 4: eine Darstellung in Längsschnitt (Aufriss) einer Ausführungsform eines Schmierstoffgebers mit Erneuerbarkeit der elektrischen Elemente,
- Fig. 5: eine Darstellung des Grundrisses des äusseren stopfenförmigen Bauteils eines Schmierstoffgebers mit Erneuerbarkeit der elektrischen Elemente,
- Fig. 6: eine Darstellung des Grundrisses des inneren stopfenförmigen Bauteils eines Schmierstoffgebers mit Erneuerbarkeit der elektrischen Elemente,
- Fig. 7: eine Darstellung des Grundrisses des Behälters, Behälterbodenseite, eines Schmierstoffgebers mit Erneuerbarkeit der elektrischen Elemente,
- Fig. 8: einen schematischen Längsschnitt einer Ausführungsform der Vorrichtung für kleine Dimensionen ("Patrone"),
- Fig. 9: einen schematischen Querschnitt einer Ausführungsform der inneren Partie der Vorrichtung für kleine Dimensionen ("Patrone"),
- Fig. 10: einen Längsschnitt einer Ausführungsform einer Infusionsspritze,
- Fig. 11: einen Querschnitt einer Ausführungsform einer Infusionsspritze,
- Fig. 12: einen Längsschnitt einer weiteren Ausführungsform einer Infusionsspritze,
- Fig. 13: einen Längsschnitt (Aufriss) einer Wegmess-Einrichtung zu einer Infusionsspritze,
- Fig. 14: einen Grundriss einer Wegmess-Einrichtung zu einer Infusionsspritze.

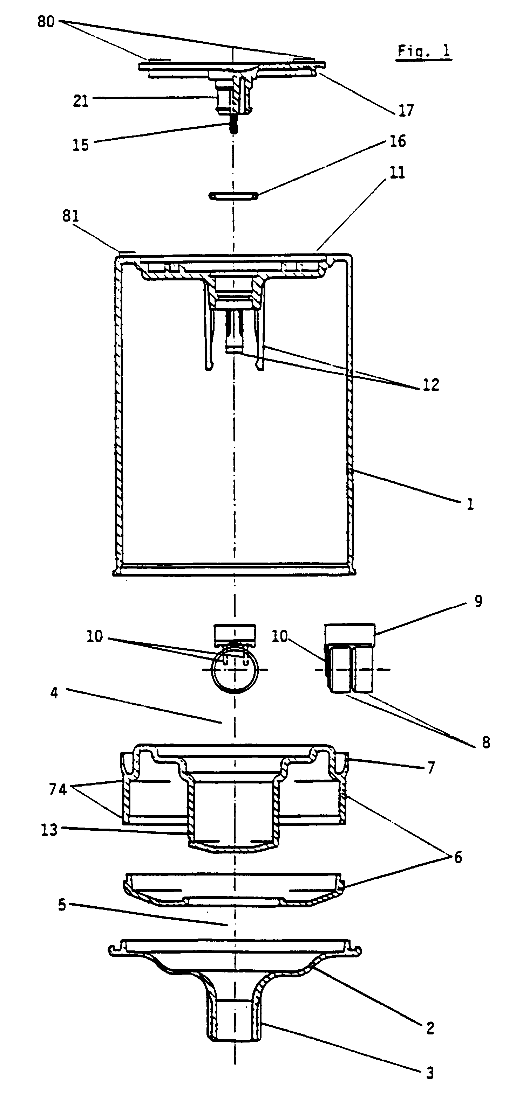

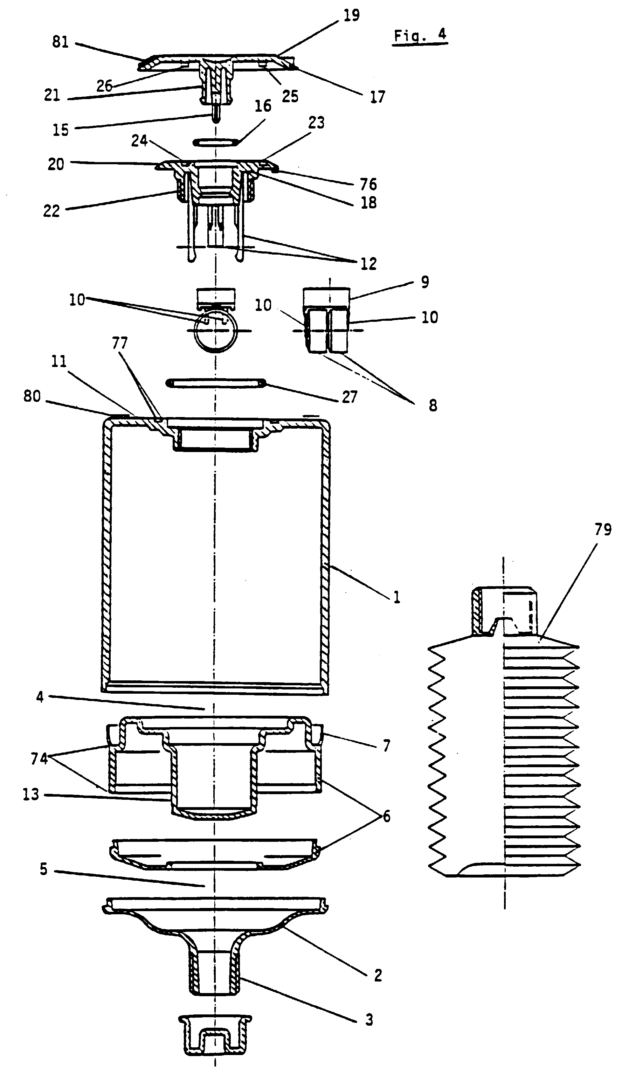

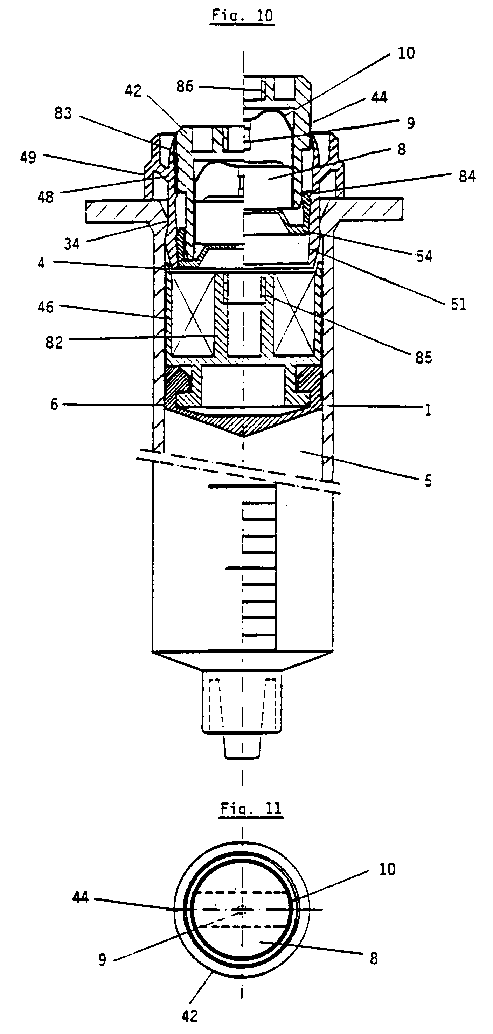

Fig. 1 ist eine schematische Darstellung in Längsschnitt (Aufriss) des prinzipiellen Aufbaus der Vorrichtung, gleichzeitig Darstellung einer Ausführungsform eines Schmierstoffgebers. 1 ist ein im wesentlichen glatter zylindrischer Behälter aus einem formstabilen Kunststoff, der einen mehrfach abgesetzten Behälterboden 11 besitzt. Letzterer ist - wie im vorliegenden Fall - mit dem Behälter 1 monolithisch fest verbunden oder von ihm getrennt, auch mehrfach geteilt ausgeführt (letzteres vergl. Fig.4). Im vorliegenden Fall trägt der Behälterboden 11 die mit ihm monolithisch fest verbundene, in das Innere des Behälters 1 vorkragende Klemmeinrichtung 12 für die elektrischen Elemente (Zelle, Widerstand, Kontakte), die aus zwei aufeinander orthogonal stehenden Paaren elastisch federnder, mit Wülsten versehenen Zungen besteht. Am Ende des Behälters 1 befindet sich der mit diesem über eine dichte Verbindung fest
verbundene Behälterabschluss 2 mit - im vorliegenden Fall - Schraubanschluss 3 (über Steckanschluss siehe Fig. 10). Der - im vorliegenden Fall - aus zwei ineinander dicht steckbaren Teilen aus elastischem Kunststoff bestehende Kolben 6 weist eine zusammen mit der Kolbengleitfläche einen Raum 74 für einen Fettfilm bildende, radial vorkragende ringförmige Dichtung 7 in Form einer Lippe und eine topfartige Ausnehmung/Vertiefung 13 auf. Durch den Kolben 6 wird der Behälterinhalt in einen Druckgasraum 4 und einen Masseraum 5 unterteilt. Die Ausnehmung/ Vertiefung 13 ist für die Kleinhaltung des Druckgasraums 4 im Interesse einer möglichst kurzen Anlaufzeit für den Kolben 6 von wesentlicher Bedeutung. Die in die Klemmeinrichtung 12 einsetzbaren elektrischen Elemente sind die Gasentwicklungszelle 8 (im vorliegenden Fall Doppelzelle), der Einstell- und Belastungswiderstand 9 (hier Potentiometer) und der Kontakt 10. Im Behälterboden 11 befindet sich das an seinem Flansch mit einer Skala 80 und an seinem rohrartigen Hals 21 mit einem nach innen vorkragenden profilierten Zapfen 15 versehene äussere steckbare stopfenförmige Bauteil 17 in Form einer drehbaren Scheibe zur Einstellung des Widerstandes 9. Dabei greift der - im vorliegenden Fall kreuzförmigen Querschnitt aufweisende - Zapfen 15 in eine entsprechende Oeffnung des Drehwiderstandes 9 ein. 16 ist ein O-Ring zur Dichtung zwischen 11 und 17. Der Uebersichtlichkeit halber sind die Einzelteile der hier vorliegenden Vorrichtung auseinandergezogen dargestellt. Diese Darstellung lässt auch die Montagereihenfolge erkennen.

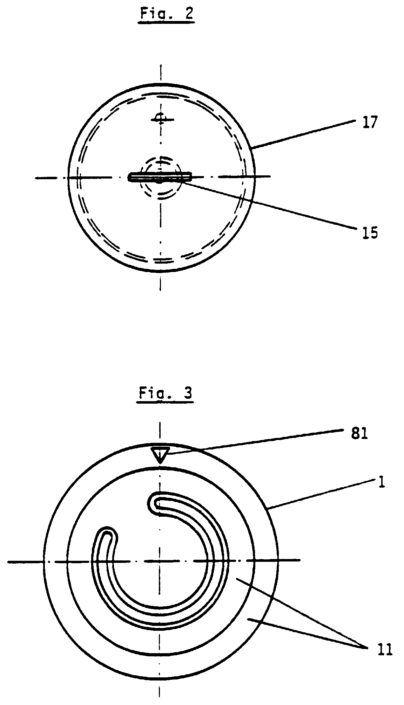

Fig. 2 zeigt eine Darstellung des Grundrisses des äusseren steckbaren stopfenförmigen Bauteils zur Einstellung des Belastungswiderstandes eines Schmierstoffgebers. Das Bauteil 17 besitzt auf der Unterseite seines Flansches einen Anschlagnocken, der in eine kreisförmige Nut des Behälterbodens 11 eingreift, die nicht einen vollen Kreisumfang umfasst sondern in entsprechenden Anschlägen endet. Damit sind die Endlagen der Einstellung des Widerstandes bestimmt. 15 ist der kreuzförmige Zapfen im Profil. Auf der oberen Seite seines Flansches weist das Bauteil 17 einen radialen Betätigungsschlitz auf.

In Fig. 3 ist eine Darstellung des Grundrisses des Behälters, Behälterbodenseite, eines Schmierstoffgebers gezeigt. 1 ist der Behälter, 11 der Behälterboden, der eine Nut mit Anschlägen aufweist, in die der Anschlagnocken des Bauteils 17 eingreift. 81 ist der Markierpfeil zur Skala 80 (vergl. Fig. 1).

Fig. 4 bezieht sich auf eine Darstellung in Längsschnitt (Aufriss) einer Ausführungsform eines Schmierstoffgebers mit Erneuerbarkeit der elektrischen Elemente. Die Bezugszeichen 1, 2, 3, 4, 5, 6, 7, 8, 9, 10, 12, 13, 74 entsprechen in Form und Funktion genau denjenigen der Fig. 1. Der Behälterboden 11 ist geteilt ausgeführt und besteht aus dem eigentlichen abgesetzten Behälterboden 11 mit einer mit Gewinde versehenen zentralen Oeffnung sowie dem äusseren steckbaren und inneren schraubbaren stopfenförmigen Bauteil 17 und 18. Der Behälterboden 11 ist mit einer ringförmigen Nut 77 und einer Skala 80 versehen. 17 ist ein drehbares Bauteil während 18 über ein Gewinde fest mit dem eigentlichen Behälterboden 11 verschraubt ist. 19 ist der scheibenartige, einen Markierungspfeil 81 zur Skala 80 tragende Flansch des äusseren Bauteils 17, während 21 dessen rohrartigen Hals und 15 den profilierten Zapfen darstellt. Das innere Bauteil 18 weist einen scheibenartigen Flansch 20 und einen rohrartigen Hals 22 sowie die Klemmeinrichtung 12 für elektrische Elemente auf. Der Flansch 20 trägt auf seiner Innenseite den peripheren Nocken 76 zur Fixierung der End stellung. Ferner sind in den Flansch 20 stirnseitig je eine äussere ringförmige Nut 23 und eine innere ringförmige Nut 24 eingearbeitet, in welche der äussere Nocken 25 bzw. der innere Nocken 26 des äusseren Bauteils 17 eingreifen. 27 ist der O-Ring zwischen Behälterboden 11 und innerem Bauteil 18, 16 derjenige zwischen letzterem und äusserem Bauteil 17. Als Zubehör ist eine mit Faltenbalg und Innengewindestutzen ausgerüstete Nachfülleinrichtung 79 in Form einer Patrone vorgesehen. Der Uebersichtlichkeit halber sind die Einzelteile auseinandergezogen dargestellt.

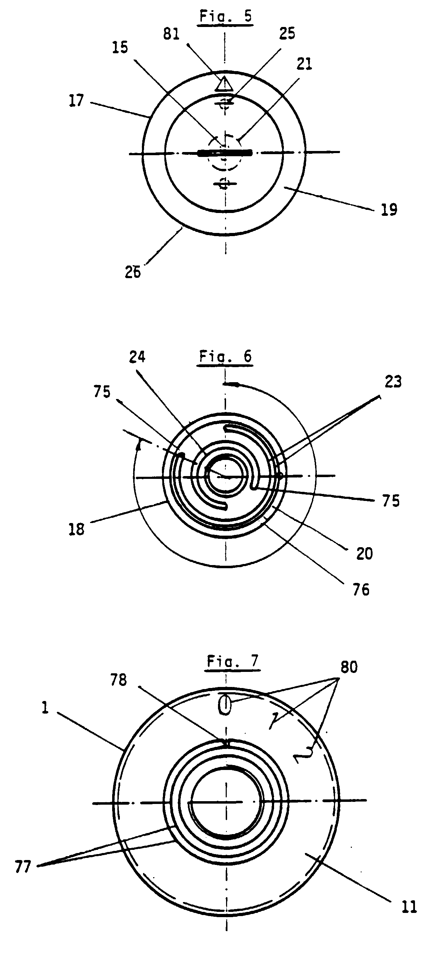

Fig. 5 zeigt eine Darstellung des Grundrisses des äusseren stopfenförmigen Bauteils eines Schmierstoffgebers mit Erneuerbarkeit der elektrischen Elemente. 17 ist das äussere steckbare stopfenförmige drehbare Bauteil, dessen scheibenartiger Flansch 19 auf seiner Aussenseite einen dreieckigen Markierpfeil 81 zur Skala trägt. Der Flansch 19 ist ausserdem mit einem zentralen radialen Betätigungsschlitz versehen. Auf der Innenseite von 19 befinden sich die Nocken 25 und 26 (gestrichelt gezeichnet). Ferner ist der profilierte Zapfen 15 sowie der rohrartige Hals 21 (gestrichelt) angedeutet.

Fig. 6 ist eine Darstellung des Grundrisses des inneren stopfenförmigen Bauteils eines Schmierstoffgebers mit Erneuerbarkeit der elektrischen Elemente. 18 stellt das innere steckbare stopfenförmige Bauteil mit seinem scheibenartigen Flansch 20 dar, in dessen Aussenseite die äussere ringförmige Nut 23 und die innere ringförmige Nut 24 eingearbeitet sind. Die Nuten 23 und 24 machen jeweils keinen ganzen Kreisumfang aus, sondern enden in entsprechende halbrunde Anschläge 75 zur Bewegungsbegrenzung des drehbaren äusseren Bauteils 17. Der maximal erreichbare Drehwinkel ist durch einen Kreis mit Pfeilspitzen angedeutet.

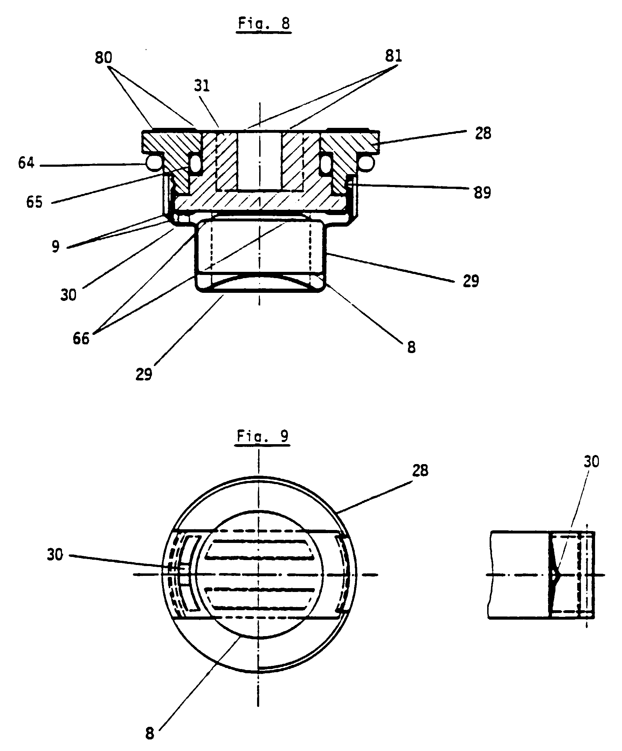

Fig. 7 zeigt eine Darstellung des Grundrisses des Behälters eines Schmierstoffgebers mit Erneuerbarkeit der elektrischen Elemente. 1 ist der Behälter, 11 der eigentliche Behälterboden mit der ringförmigen Nut 77 mit Anschlag 78 und seiner zentralen, mit einem Gewinde versehenen Oeffnung zur Aufnahme des inneren schraubbaren stopfenförmigen Bauteils 18. Der Behälterboden 11 trägt eine Skala 80 zur Kennzeichnung der relativen Stellung des Flansches 19 des äusseren stopfenförmigen drehbaren Bauteils 17.

In Fig. 8 ist ein schematischer Längsschnitt einer Ausführungsform der Vorrichtung für kleine Dimensionen ("Patrone") dargestellt. Behälter und Kolben sind in dieser Figur der Einfachheit halber weggelassen worden. Der hohlzylindrische Behälter weist keinen eigentlichen Behälterboden auf sondern endet in Form eines offenen Rohrstücks. Die Stelle und die Funktion des Behälterbodens übernehmen das äussere topfartige Bauteil 28 und das innere, als Drehknopf ausgebildete topfartige Bauteil 31. Das auf seiner Stirnseite eine Skala 80 aufweisende äussere Bauteil 28 ist durch eine Steck- oder Schraubverbindung mit dem hohlzylindrischen Ende des Behälters verbunden (nicht gezeichnet). Mit 28 ist über eine Kerbe eine einen Einrastwulst 89 enthaltende, radial stehende elastische Halte- und Federklammer 29 verbunden. Die Halte- und Federklammer 29 dient zur Halterung der Gasentwicklungszelle 8. Der axial angeordnete Kontakt 30 (Schleifkontakt) ist in der Halte- und Federklammer 29 integriert. Das innere topfartige Bauteil 31 trägt auf seiner inneren Stirnseite den ringförmigen Einstellwiderstand 9 (Potentiometer) und den mit diesem fest verbundenen zentralen Kontakt 66, der den Stromkreis zur Zelle 8 vervollständigt. 31 ist auf seiner Stirnseite mit einem Markierpfeil 81 zur Skala versehen. Zwischen Behälter und äusserem topfartigen Bauteil 28 und zwischen letzterem und innerem topfartigen Bauteil 31 befindet sich je ein O-Ring 64 bzw. 65 als Dichtung.

In Fig. 9 ist ein schematischer Querschnitt einer Ausführungsform der inneren Partie der Vorrichtung für kleine Dimensionen ("Patrone") dargestellt. 28 ist die Kontur der abgesetzten Partie des äusseren topfartigen Bauteils. 30 stellt den axial angeordneten Kontakt mit radialer Kontaktzunge (Schleifkontakt) dar. Dieser Kontakt 30 ist in der Nebenfigur rechts im Bild im Seitenriss nochmals wiedergegeben. 8 ist die zylindrische, tablettenförmige Einzel-Gasentwicklungszelle.

Fig. 10 ist ein Längsschnitt einer Ausführungsform einer Infusionsspritze. Form und Dimensionen des Behälters 1 entsprechen denjenigen einer normierten handelsüblichen graduierten Infusionsspritze mit konischem Steckanschluss zur Aufnahme einer Injektionsnadel oder eines Katheters. Der Behälter 1 weist an seinem flanschseitigen Ende einen nach innen vorspringenden Wulst 34 auf. 4 ist der Druckgasraum, 5 der Masseraum zur Aufnahme der einzuflössenden Flüssigkeit und 6 ein mehrteiliger Kolben mit innenliegendem Kolbenkern 82 mit Gewinde 85 für eine Betätigungsstange zum Aufziehen des Mediums. Der Kolben weist ausserdem fahnenförmige Führungsrippen 46 auf. 48 ist eine aussen liegende radial-elastische Hülse, deren Anschlagring 49 auf dem Flansch des Behälters 1 aufliegt. Die aussen liegende Hülse 48 weist eine radial nach innen federnde Dichtungslippe 83 und mehrere innen liegende konische Absetzungen 51 sowie einen nach innen vorspringenden Anschlagwulst 84 auf. In der Hülse 48 steckt ein innen liegender hohler Press/Dichtungs-Stopfen 42, der auf seiner äusseren Mantelfläche mindestens eine axial verlaufende Entlüftungsöffnung 44 in Form eines schmalen Schlitzes sowie an seinem äusseren Ende ein Gewinde 86 für eine Betätigungsstange zur Demontage enthält. Zwischen Hülse 48 und Stopfen 42 befindet sich ein axial/radialelastischer Verriegelungs/Einrast-Ring 54, der zugleich der Halterung der Gasentwicklungszelle 8 und der kraftschlüssigen Fixierung des festen stabförmigen Widerstandes 9 und des U-förmigen federnden Kontaktes 10 dient. In der linken Bildhälfte ist die normale Betriebsstellung, in der rechten Bildhälfte die Ausgangsstellung vor Inbetriebsetzung dargestellt.

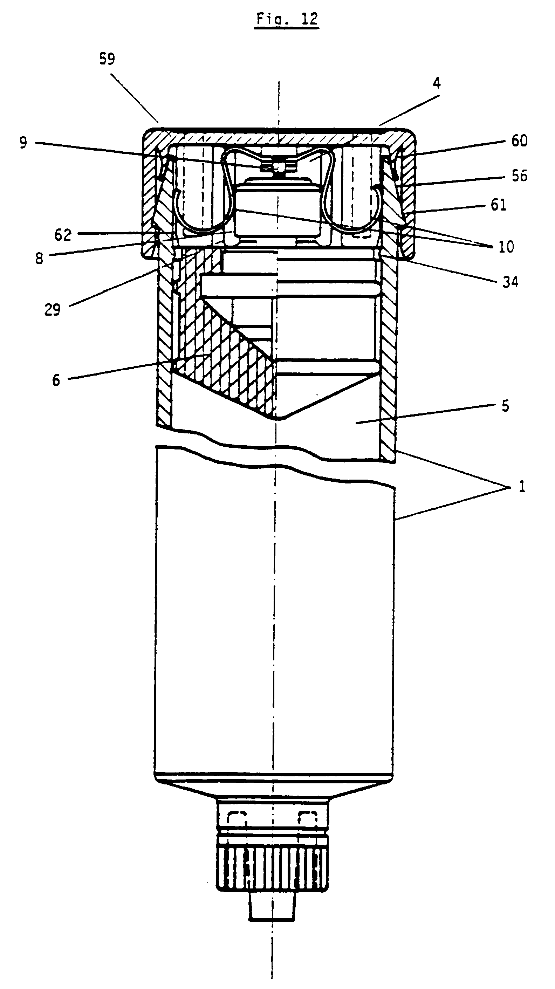

Fig. 11 zeigt einen Querschnitt einer Ausführungsform einer Infusionsspritze. Der innen liegende hohle Press/ Dichtungs-Stopfen 42 weist an seinem äusseren Umfang längs der Mantellinien verlaufende um 90° gegeneinander versetzte, axial verlaufende Entlüftungsöffnungen 44 in Form von leichten Kerben auf. 8 ist die Gasentwicklungszelle, 9 der (gestrichelt gezeichnete) stabförmige Widerstand vom Profil her gesehen und 10 der U-förmige federnde Kontakt, der sich mit seinen Schenkeln der Innenkontur des zylindrischen Hohlraums des Stopfens 42 anschmiegt.

In Fig. 12 ist ein Längsschnitt einer weiteren Ausführungsform einer Infusionsspritze dargestellt. 1 ist der Behälter einer herkömmlichen handelsüblichen graduierten Infusionsspritze mit konischem Steckanschluss. 4 stellt den Druckgasraum und 5 den Masseraum dar. 6 ist ein meist mehrteilig ausgeführter Kolben. Der Behälter 1 besitzt an seinem offenen Ende einen nach innen vorspringenden Wulst 34 als Sicherheitsanschlag für den Kolben 6 und eine aussen liegende konische Absetzung 56. Die Funktion des Behälterbodens übernimmt eine als Abschlussdeckel dienende radial elastische Kappe 59, die an ihrer innen liegenden Ecke eine radial nach innen wirkende federnde Dichtungslippe 60 und eine innen liegende konische Eindrehung 61 aufweist. In letztere rastet die Kante der konischen Absetzung 56 des Behälters 1 ein. Die elastische Kappe 59 ist in ihrer Mittelpartie ausserdem mit radial stehenden, radial nach innen und axial nach aussen wirkenden hakenförmigen Halte- und Federklammern 29 zur Halterung der Gasentwicklungszelle 8 und in ihrer weiter aussen liegenden Partie mit axialen Führungsrippen 62 zur Halterung der doppel-S-förmigen Kontakte 10 ausgerüstet. 9 ist der feste Belastungswiderstand.

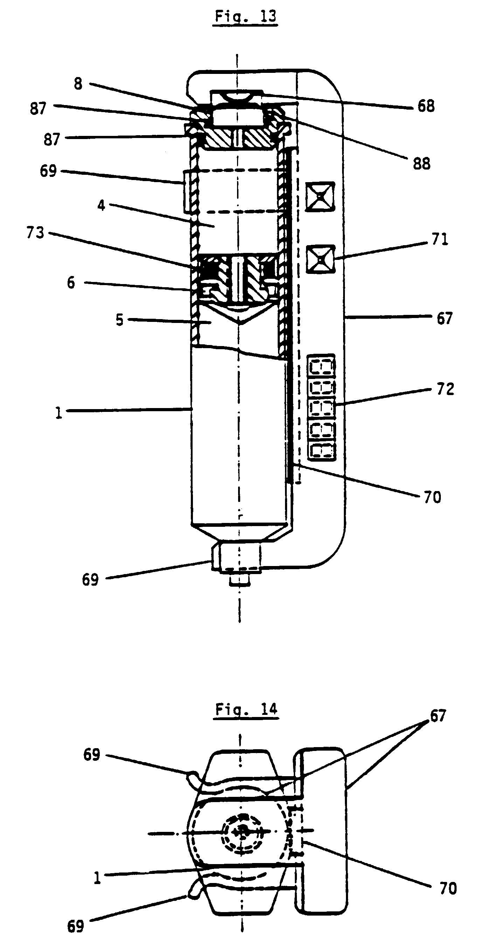

In Fig. 13 ist ein Längsschnitt (Aufriss) einer Wegmess-Einrichtung zu einer Infusionsspritze dargestellt. 1 ist der Behälter mit dem Druckgasraum 4 und dem Masseraum 5. 67 ist ein U-förmiges Gehäuse, das an seiner einen Stirnseite einen axialen Anschlagkontakt 68 und in den übrigen Partien mehrere Klemmbacken 69 zum Festhalten der Infusionsspritze sowie eine lineare Spule 70, Tastknöpfe 71 und eine Anzeige 72 besitzt. Zwischen der tablettenförmigen Gasentwicklungszelle 8 und dem Gehäuse 67 ist ferner ein radialer Kontakt 88 angeordnet. Im übrigen sind die üblichen Dichtungen 87 zum Abschliessen des Gasraumes 4 vorgesehen. Der Kolben ist mit einem Dauermagneten 73 ausgerüstet, der über seine elektromagnetische Kopplung mit der linearen Spule die Kolbenstellung und damit den Bewegungsmechanismus des Kolbens steuert.

Fig. 14 zeigt einen Grundriss der Wegmess-Einrichtung zu einer Infusionsspritze. Die Bezugszeichen 1, 67, 69 und 70 entsprechen genau denjenigen der Fig. 13 und bedürfen keiner weiteren Erklärung. Es soll noch auf die raumsparende Ausführung dieser Apparatur hingewiesen werden.

### Ausführungsbeispiel 1:

### Siehe Figuren 4, 5, 6 und 7!

Die Vorrichtung ist als im Betrieb wartungsfreier automatischer Schmierstoffgeber mit Austauschbarkeit und Auswechselbarkeit der elektrischen Elemente sowie Möglichkeit des Nachfüllens von Schmierstoff ausgebildet. Der Schmierstoffgeber besteht grundsätzlich aus dem hohlzylindrischen Behälter 1 aus durchsichtigem formbeständigen Kunststoff, dem Behälterabschluss 2 mit Schraub- oder Steckanschluss 3, dem Kolben 6, dem äusseren steckbaren stopfenförmigen Bauteil 17, dem inneren steckbaren stopfenförmigen Bauteil 18 und den elektrischen Elementen 8, 9 und 10. Im vorliegenden Fall ist der Behälterabschluss 2 aus elastischem Kunststoff mit gewelltem Trichter zwecks Nachgiebigkeit bei Schlag- und Vibrationsbeanspruchung ausgeführt. Der Kolben 6 ist aus zwei Teilen zusammengesetzt und weist am druckraumseitigen Ende eine im Durchmesser vorgespannte elastische Dichtungslippe 7 auf, die sich im Betrieb an die Innenwand des Behälters 1 anschmiegt. Die Dichtungslippe 7 begrenzt zusammen mit der im Durchmesser kleineren zylindrischen Gleitfläche den Raum 74 für den Fettfilm, wodurch ideale Dichtungs- und Schmierungsverhältnisse für den Kolben 6 geschaffen werden. Letzterer besitzt eine topfartige Ausnehmung/Vertiefung 13 zur Minimalisierung des toten Volumens im Druckgasraum 4 und zur Erreichung einer möglichst kurzen Anlaufzeit des Kolbens 6 nach Inbetriebsetzung. Der Behälterboden 11 trägt die Skala 80 und ist mit der Nut 77 mit Anschlag 78 versehen. Das aus formstabilem festem Kunststoff bestehende innere Bauteil 18 weist an seinem rohrartigen Hals 22 im vorliegenden Fall ein Gewinde auf, mit dem es in ein entsprechendes Innengewinde in der Oeffnung des Behälterbodens 11 eingeschraubt wird. Die zur Aufnahme der elektrischen Elemente, Gasentwicklungszelle 8, Drehwiderstand (Potentiometer) 9 und Kontakte 10 dienende Klemmeinrichtung 12 besteht aus zwei Paaren federnder zungenartiger Greifer, welche im wesentlichen die Doppelzelle 8 in allen Richtungen festhalten. Die elektrischen Elemente 8, 9, 10 sind im vorliegenden Fall fest zu einer kompakten Einheit miteinander verschweisst. Dies hat den Vorteil, dass bei Demontage, Auswechslung etc. keine Kontakt- und Verschmutzungsprobleme auftauchen. Die Elemente 8, 9, 10 können als Variante jedoch auch in einer gemeinsamen Kassette untergebracht sein. Das äussere stopfenförmige Bauteil 17 ist steckbar ausgeführt und greift mit seinem elastischen rohrartigen Hals 21 in eine entsprechende abgesetzte Bohrung im inneren Bauteil 18 ein. Der profilierte Zapfen 15 zur Uebertragung der Drehbewegung auf das Potentiometer 9 hat im vorliegenden Fall den Querschnitt eines Kreuzes (12 Ecken). Die Nocken 25 und 26 des äusseren Bauteils 17 greifen in entsprechende ringförmige Nuten 23 und 24 des inneren Bauteils 18 mit ihren Anschlägen 75 ein, wodurch die Drehbewegungen begrenzt sind (siehe Figurenbeschreibung). Der periphere Nocken 76 des inneren Bauteils 18 bewegt sich in der Nut 77 im Behälterboden (Fixierung der Endstellung). Durch Einstellung des mit Markierpfeil 81 versehenen äusseren Bauteils 17 gegenüber der Skala 80 auf dem Behälterboden 11 wird die Laufzeit eingestellt. Die als Faltenbalg ausgeführte Nachfülleinrichtung 79 kann zu diesem Zweck auf das Gewinde des Schraubanschlusses 3 aufgeschraubt werden.

### Ausführungsbeispiel 2:

### Siehe Figuren 8 und 9!

Es handelt sich um eine Ausführungsform der Vorrichtung für vergleichsweise kleine Dimensionen, sogenannte "Patrone". Das die kreisringförmige Skala 80 tragende äussere topfartige Bauteil 28 besteht aus einem formbeständigen Kunststoff. Es ist gegenüber dem Behälter durch den O-Ring 64 und gegenüber dem drehbaren inneren topfartigen, ebenfalls aus Kunststoff bestehenden Bauteil (Drehknopf) 31 durch den O-Ring 65 gasdicht abgedichtet. Das äussere Bauteil 28 besitzt an seinem halsartigen abgesetzten inneren Ende zwei Kerben, in die die Halte- und Federklammer 29 mittels des Einrastwulstes 89 montier- und demontierbar radial federnd einschnappt. Die Halte- und Federklammer 29 ist im Berührungsbereich mit dem äusseren Bauteil 28 segmentartig ausgeführt und die Segmente sind in letzterem eingelassen. Vorzugsweise besteht die Halte- und Federklammer 29 aus korrosionsbeständigem gut federnden metallischen Werkstoff. Der als axiale Feder zur Halterung der Gasentwicklungszelle 8 dienende Boden von 29 ist nach innen gewölbt. Zur Auswechslung und Erneuerung der Gasentwicklungszelle 8 wird die Halte- und Federklammer 29 von Hand axial vom äusseren topfartigen Bauteil 28 abgezogen, die verbrauchte Zelle 8 herausgenommen, eine neue eingesetzt und das Ganze wieder axial auf das Bauteil 28 aufgesteckt. Die gewünschte Gasmenge pro Zeiteinheit wird durch Verdrehen des inneren topfartigen Bauteils 31 eingestellt, wobei die relative Stellung des Markierpfeils 81 gegenüber der Skala 80 auf der Stirnseite des äusseren topfartigen Bauteils 28 ein Mass für die Gasproduktion ist. Dem Betrieb bei unterschiedlichen Temperaturen wird dadurch Rechnung getragen, dass die Skala 80 mit verschiedenen Graduierungen ausgeführt wird.

### Ausführungsbeispiel 3:

### Siehe Figuren 10 und 11!

Die Vorrichtung stellt im vorliegenden Fall ein selbsttätig arbeitendes Infusionsgerät dar, welches im allgemeinen eine pro Zeiteinheit konstante Menge einer bestimmten Infusionslösung an den Patienten abgibt. In der Regel wird zu diesem Zweck als Behälter 1 das Gefäss einer handelsüblichen normierten hohlzylindrischen Infusionsspritze verwendet. In das Gewinde 85 im Kolbenkern 82 wird eine aus Metall oder Kunststoff bestehende Betätigungsstange (Kolbenstange) eingeschraubt und mit ihrer Hilfe das zu verabreichende Medium durch Kolbenbewegung bis zum als Anschlag dienenden, nach innen vorspringenden Wulst 34 des Behälters 1 aufgezogen. Die aus nachgiebigem Kunststoff bestehende aussen liegende radial-elastische Hülse 48 wird zusammen mit dem aus formbeständigem Kunststoff gefertigten innen liegenden hohlen Press/Dichtungs-Stopfen 42 inklusive elektrische Elemente 8, 9, 10 axial in den Behälter 1 eingepresst, bis der Anschlagring 49 auf den Flansch des Behälters 1 aufzuliegen kommt. Durch den in die innen liegende konische Absetzung 51 der radial-elastischen Hülse 48 einrastenden Verriegelungs/Einrastring 54 wird letztere radial nach aussen gedrückt und hinter den nach innen vorspringenden Wulst 34 des Behälters 1 festgeklemmt und gasdicht abgedichtet: Linke Bildhälfte! Beim Einschieben des Stopfens 42 wird die Gasentwicklungszelle 8 über den Widerstand 9 eingeschaltet, wobei gleichzeitig eine Vorkompression des Gases (Luft) im Druckgasraum 4 erzielt wird, so dass die Anlaufzeit des Kolbens 6 verkürzt wird. Die Gasentwicklungszelle 8 wird teils durch Haftreibung, teils durch Federspannung über Kontakt 10 festgehalten. Zwecks Demontierung und Auswechslung der Gasentwicklungszelle 8 wird die oben erwähnte Betätigungsstange in das Gewinde 86 des Press/Dichtungs-Stopfens 42 eingeschraubt und letzterer axial herausgezogen, bis die elektrischen Elemente 8, 9, 10 zugänglich sind und die alte Gasentwicklungszelle entfernt und durch eine neue ersetzt werden kann. Bei unzulässigem Ueberdruck im Druckgasraum 4 wird der innen liegende Press/ Dichtungsstopfen 42 unter Ueberwindung seiner Haftreibung im Normalbetrieb axial nach aussen gepresst, bis seine axialen Entlüftungsöffnungen 44 über die Höhe der Dichtungslippe 83 der aussen liegenden radial-elastischen Hülse 48 zu stehen kommen, wobei der Verriegelungs/Einrastring 54 in seiner Lage stehen bleibt und als axiale Führung des innen liegenden hohlen Press/Dichtungsstopfens 42 dient. Die Gasentwicklungszelle 8 wird dabei dank ihrer radialen Haftreibung mit dem Kontakt 10 zusammen mit dem Stopfen 42 nach aussen verschoben, ohne dass ihre Gasentwicklung unterbrochen wird. Damit ist der Weg für die Druckentlastung über die Entlüftungsöffnungen 44 freigelegt. Im Falle der äusseren mechanischen Blockierung des Stopfens 42 kann der Ueberdruck über die radial vorgespannten Dichtungslippen 83 durch deren Aufweitung sicher abgebaut werden: Doppelte Sicherheit! Nach erfolgtem Druckabbau kann die Normalbetriebslage durch manuelles axiales Hineindrücken des Press/Dichtungs-Stopfens 42 und damit die betriebsmässige Förderung des Mediums wieder erstellt werden.

Die Vorrichtung zur gezielten, steuerbaren Abgabe einer Flüssigkeit oder einer viskosen Masse oder einer Suspension von Feststoffpartikeln in einer Flüssigkeit besteht aus einem zylindrischen Behälter 1, einem Behälterabschluss 2 mit Schraub- oder Steckanschluss 3, einem in den Behälter 1 mit Schiebesitz passenden, letzteren in einen Druckgasraum 4 und einen Masseraum 5 unterteilenden zylindrischen Kolben 6 mit ringförmiger Dichtung 7 am Umfang, einem die elektrischen Elemente wie elektro-chemische Gasentwicklungszellen 8, Einstell- und Belastungswiderstände 9 und Kontakte 10 enthaltenden Bauteil, wobei die Gasentwicklungszelle 8 tablettenförmige, allseitig metallisch abgeschlossene Bauart aufweist, eine Gasdiffusionselektrode, einen wässrigen Elektrolyten und eine Gegenelektrode besitzt und durch Schliessung eines äusseren Stromkreises aktivierbar ist, das die elektrischen Elemente (8;9;10) enthaltende Bauteil der mit dem Behälter 1 eine monolithische Struktur bildende oder mit dem letzteren über eine lösbare Schraub- oder Steckverbindung verbundene Behälterboden 11 ist, wobei die elektrischen Elemente (8; 9; 10) durch eine Klemmeinrichtung 12 festgehalten sind und am masseraumseitigen Ende des Behälters 1 ein trichterförmiger Behälterabschluss 2 vorgesehen ist. Der Kolben 8 ist auf der Druckgasraumseite vorteilhafterweise mit einer einen Raum 74 für einen Fettfilm schaffenden vorkragenden ringförmigen Dichtungslippe 7 und im zentralen Teil mit einer den Druckgasraum 4 auf ein Minimum reduzierenden topfartigen Ausnehmung/Vertiefung 13 versehen und der Behälterabschluss 2 ist in Form eines trichterförmigen elastischen gewellten Abschlussdeckels mit Gewindenippel ausgeführt, wobei die als Doppelzelle mit elektrischem Widerstand in Kompaktbauweise ausgeführten elektrischen Elemente (8; 9; 10) über eine Klemmeinrichtung 12 unmittelbar im mit Markierpfeil 81 zu Skala versehenen Behälterboden 11 gehalten sind, und ferner im Behälterboden 11 ein mit Skala 80 ausgerüstetes und mit O-Ring 16 abgedichtetes, einen rohrartigen Hals 21 aufweisendes drehbares stopfenförmiges Bauteil 17 zur Einstellung des als Dreh-Potentiometer ausgeführten elektrischen Widerstandes 9 vorgesehen ist, das einen profilierten Zapfen 15 besitzt, der in eine entsprechende Oeffnung des Dreh-Potentiometers mit einem Schiebesitz eingreift.

Die als Doppelzelle mit elektrischem Widerstand in Kompaktbauweise ausgeführten elektrischen Elemente (8; 9; 10) sind vorzugsweise über eine Klemmeinrichtung 12 mittelbar im mit Skala 80 versehenen Behälterboden 11 gehalten, wobei letzterer zwecks Auswechselbarkeit und Erneuerbarkeit der elektrischen Elemente (8; 9; 10) und Nachfüllbarkeit des zu fördernden Mediums mehrfach unterteilt ausgeführt ist, wobei er im zentralen Teil mindestens zwei rotationssymmetrische, je einen scheibenartigen Flansch (19; 20) und einen Hals (21; 22) aufweisende demontierbare, mit durch Anschlag 75 begrenzte Nut (23; 24) und Nocken (25; 26) versehene, koaxial ineinander steckbare Bauteile (17; 18) mit Schnapp- und Greifmechanismen für die gegenseitige Fixierung und Halterung der Gasentwicklungszellen 8 und der als Dreh-Potentiometer ausgeführten Widerstände 9 enthält, indem mindestens eines der steckbaren Bauteile 17 mit einem profilierten Zapfen 15 ausgerüstet ist, der in eine entsprechende Oeffnung des Dreh-Potentiometers mit einem Schiebesitz eingreift. Vorzugsweise ist ein erstes inneres, mit einer Klemmeinrichtung 12 mit nach innen gerichteten federnden Zungen zur Halterung der zylindrischen knopfförmigen Gasentwicklungszellen 8 und des drehbaren Einstellwiderstandes 9 versehenes rotationssymmetrisches stopfenförmiges Bauteil 18 mit einem scheibenartigen Flansch 20 mit peripherem, in eine Nut 77 mit Anschlag 78 des Behälterbodens 11 eingreifenden Nocken 76 und einem rohrartigen Hals 22 mit Aussengewinde vorhanden, welches in ein entsprechendes Innengewinde in einer zentralen Oeffnung des abgesetzten Behälterbodens 11 einschraubbar ist, ferner ist ein zweites äusseres ebenfalls stopfenförmiges mit Markierungspfeil 81 zu Skala 80 versehenes Bauteil 17 vorhanden, das mittels eines rohrartigen, radial nachgiebigen Schnappverschlusses ins erste steckbar gestaltet ist, wobei sowohl zwischen dem Behälterboden 11 über eine kreisförmige Vertiefung und dem ersten Bauteil 18 wie auch zwischen diesem ebenfalls über eine Vertiefung und dem zweiten Bauteil 17 je ein O-Ring (27; 16) als Dichtung angeordnet ist und ausserdem eine Nachfülleinrichtung 79 in Form einer faltenbalgartigen Patrone mit Innengewindeansatz vorgesehen ist.

In einer bevorzugten Ausführung ist der die elektrischen Elemente (8; 9; 10) enthaltende Behälterboden 11 geteilt ausgeführt, wobei ein äusseres, mit den benachbarten Bauelementen durch O-Ringe (64; 65) abgedichtetes, mit dem zylindrischen Behälterende verschraubbares, eine Skala 80 aufweisendes topfartiges Bauteil 28 mit einer über einen Einrastwulst 89 aufgeklemmten mit radial nach innen und axial nach aussen wirkender, zentral-symmetrisch angeordneter Halte- und Federklammer 29 zur Halterung der Einzelzelle 8 sowie ein axial angeordneter Kontakt 30 mit radialer Kontaktzunge vorhanden ist, während ein inneres, in das äussere Bauteil 28 einpressbares, einen Markierpfeil 81 zur Skala aufweisendes rotationssymmetrisches Bauteil 31 einen kreisringförmigen Einstellwiderstand 9 und einen zentralen, mit diesem fest verbundenen Kontakt 66 trägt.

In einer Variante der Vorrichtung entspricht der zylindrische Behälter 1 dem graduierten Gefäss einer standardisierten Infusionsspritze und besitzt am bodenseitigen Ende einen nach innen vorspringenden Wulst 34, wobei ein aus mehreren Teilen bestehender Kolben 6 mit einem Kolbenkern 82 mit Gewinde 85 für Betätigungsstange zum Aufziehen des Mediums sowie mit radial stehenden fahnenförmigen Führungsrippen 46 vorgesehen ist, und wobei das den Behälterboden 11 bildende, die elektrischen Elemente (8; 9; 10) enthaltende Bauteil aus einer aussen liegenden, mit Anschlagring 49, stirnseitiger Dichtungslippe 83, nach innen vorspringendem Anschlagwulst 84 und mehreren innen liegenden konischen Absetzungen 51 versehenen rotationssymmetrischen radial elastischen Hülse 48 und einem innen liegenden, an seinem offenen Ende mit einem radial/ axial federnden inneren Verriegelungs/Einrast-Ring 54 sowie Entlüftungsöffnungen 44 zur Druckentlastung versehenen rotationssymmetrischen hohlen Press/Dichtungs-Stopfen 42 besteht, der in seiner äusseren Stirnseite ein Innengewinde 86 für Betätigungsstange zur Demontage trägt, und schliesslich die elektrischen Elemente (8; 9; 10) aus einer axial gelagerten zylindrischen Einzelzelle 8, einem kurzen, axial stehenden festen elektrischen Stabwiderstand 9 und aus als U/S-förmig gekrümmten Bändern gestalteten federnden Kontakten 10 bestehen.

In einer weiteren Variante entsprechend Infusionsspritze besitzt der zylindrische Behälter 1 am bodenseitigen Ende eine nach aussen vorkragende konische Absetzung 56 und einen nach innen vorspringenden Wulst 34, wobei ein Kolben 6 vorgesehen ist, und das den Behälterboden 11 bildende, die elektrischen Elemente (8; 9; 10) enthaltende Bauteil aus einer einzigen rotationssymmetrischen, im wesentlichen radial elastischen Kappe 59 als Abschlussdeckel mit einer innen liegenden federnden Dichtungslippe 60 und einer innen liegenden konischen Eindrehung 61, die in die konische Absetzung 56 des Behälters 1 eingreift, sowie mit radial nach innen und axial nach aussen wirkenden, zentral-symmetrisch angeordneten Halte- und Federklammern 29 zur Halterung der Einzelzelle 8 und mit axial stehenden Führungsrippen 62 für die bügelförmigen Kontakte 10 besteht und schliesslich die elektrischen Elemente (8; 9; 10) aus einer axial gelagerten zylindrischen Einzelzelle 8, einem kurzen, axial stehenden festen elektrischen Stab-, widerstand 9 und aus als doppel-S-förmig gekrümmte Bänder gestalteten federnden Kontakten 10 bestehen.

In einer besonderen Ausführung ist der mit Dichtung 87 abgeschlossene zylindrische Behälter 1 mit einer aus einem U-förmigen Gehäuse 67, einem axialen Anschlagkontakt 68, einem radialen Kontakt 88 zwischen Gasentwicklungszelle 8 und Gehäuse 67, Klemmbacken 69, einer linearen Spule 70, Tastknöpfen 71 und einer Anzeige 72 bestehenden Wegmess-Einrichtung mechanisch fest über die Klemmbacken 69 und den Anschlagkontakt 68 und über die Kontakte (68; 88) ausserdem elektrisch verbunden, wobei der Kolben 6 in seinem Innern mit einem auf die Spule 70 einwirkenden Dauermagneten 73 bestückt ist.

Die Wegmess-Einrichtung dient einer steuerbaren Gasproduktion sowie Beschränkung der Gasproduktion, im Grenzfall auf Null.

### Vorteile der Erfindung

### Allgemein und Schmierstoffgeber:

- Hygienisches, einfaches und praktisches Nachfüllen der zu fördernden Masse ohne Demontage des Behälterabschlusses möglich.
- Zusammenfassung der elektrischen Elemente zu einer kompakten Einheit: Keine Kontakt-, Korrosions- und Verschmutzungsprobleme.
- Einwandfreie, mit dem Kolben eine monolithische Einheit bildende Kolbendichtung ohne spezielle Dichtungselemente wie Kolbenringe etc. Selbstdichtend, selbstschmierend, ohne nachteilige Fettabstreifung.
- Geringe Kolbenreibung, daher kleine Weghysterese und geringe notwendige Anlaufkraft.
- Behälterabschluss vollelastisch, unempfindlich gegen Vibrationen und Stösse.

### Infusionsgerät:

- Verwendbarkeit normierter Infusionsspritzen erübrigt Sonderanfertigung von Behältern.
- Visuelle Ueberdruckanzeige.
- Einfache hygienische und umweltfreundliche Auswechselbarkeit der Gasentwicklungszelle.
- Doppelte Sicherung gegen unzulässig hohen Ueberdruck.
- Einfacher Aufbau multifunktionaler Bauelemente: Wegfall spezieller Dichtungs- und Fixierungselemente.

### Wegmessgerät:

- Einfache Aufgabentrennung von mechanischem Wegwerfteil und elektrischer Einrichtung (Mikroprozessor).
- Stufenlose Programmsteuerung, durch keinerlei Faktoren oder Begrenzungen eingeschränkt.
- Akustische und visuelle Warnung bei Abweichungen vom Sollbetrieb.

## Patentansprüche

1. Vorrichtung zur gezielten, steuerbaren Abgabe einer Flüssigkeit oder einer viskosen Masse oder einer Suspension von Feststoffpartikeln in einer Flüssigkeit, bestehend aus
a) einem zylindrischen Behälter (1 ) mit einem Schiebesitz für einen zylindrischen Kolben (6),
b) einem Behälterabschluß (2) mit Schraub- oder Steckanschluß (3),
c) wobei der in den Behälter (1) mit Schiebesitz passende, den Behälter (1) in einen Druckgasraum (4) und einen Masseraum (5) unterteilende zylindrische Kolben (6) mit einer Umfangsdichtung versehen ist,
d) einem die elektrischen Elemente wie elektrochemische Gasentwicklungszellen (8), Einstell- und Belastungswiderstände (9) und Kontakte (10) enthaltenden Bauteil, wobei
- die Gasentwicklungszelle (8) tablettenförmige, allseitig metallisch geschlossene Bauart aufweist, eine Gasdiffusionselektrode, einen wässrigen Elektrolyten und eine Gegenelektrode besitzt und durch Schliessung eines äußeren Stromkreises aktivierbar ist,
- das die elektrischen Elemente (8;9;10) enthaltende Bauteil der mit dem Behälter (1) eine monolithische Struktur bildende oder mit dem Behälter (1) über eine lösbare Schraub- oder Steckverbindung verbundene Behälterboden (11) ist, und
- die elektrischen Elemente (8;9;10) durch eine Klemmeinrichtung (12) festgehalten sind und am masseraumseitigen Ende des Behälters (1) ein trichterförmiger Behälterabschluß (2) vorgesehen ist,
dadurch gekenzeichnet, daß
e) die Umfangsdichtung eine radial vorgespannte Dichtungslippe (7, 83) aufweist.

2. Vorrichtung nach Anspruch 1, dadurch gekennzeichnet, dass der Kolben (6) auf der Druckgasraumseite mit einer einen Raum (74) für einen Fettfilm schaffenden vorkragenden ringförmigen Dichtungslippe (7) und im zentralen Teil mit einer den Druckgasraum (4) auf ein Minimum reduzierenden topfartigen Ausnehmung/Vertiefung (13) versehen ist und der Behälterabschluss (2) in Form eines trichterförmigen elastischen gewellten Abschlussdeckels mit Gewindenippel ausgeführt ist und dass die als Doppelzelle mit elektrischem Widerstand in Kompaktbauweise ausgeführten elektrischen Elemente (8; 9; 10) über eine Klemmeinrichtung (12) unmittelbar im mit Markierpfeil (81) zu Skala versehenen Behälterboden (11) gehalten sind, und dass ferner im Behälterboden (11) ein mit Skala (80) ausgerüstetes und mit O-Ring (16) abgedichtetes, einen rohrartigen Hals (21) aufweisendes drehbares stopfenförmiges Bauteil (17) zur Einstellung des als Dreh-Potentiometer ausgeführten elektrischen Widerstandes (9) vorgesehen ist, das einen profilierten Zapfen (15) besitzt, der in eine entsprechende Oeffnung des Dreh-Potentiometers mit einem Schiebesitz eingreift.

3. Vorrichtung nach Anspruch 1, dadurch gekennzeichnet, dass der Kolben (6) auf der Druckraumseite mit einer einen Raum (74) für einen Fettfilm schaffenden vorkragenden ringförmigen Dichtungslippe (7) und im zentralen Teil mit einer den Druckgasraum (4) auf ein Minimum reduzierenden topfartigen Ausnehmung/Vertiefung (13) versehen ist und der Behälterabschluss (2) in Form eines trichterförmigen elastischen gewellten Abschlussdeckels mit Gewindenippel ausgeführt ist und dass die als Doppelzelle mit elektrischem Widerstand in Kompaktbauweise ausgeführten elektrischen Elemente (8; 9; 10) über eine Klemmeinrichtung (12) mittelbar im mit Skala (80) versehenen Behälterboden (11) gehalten sind, wobei letzterer zwecks Auswechselbarkeit und Erneuerbarkeit der elektrischen Elemente (8; 9; 10) und Nachfüllbarkeit des zu fördernden Mediums mehrfach unterteilt ausgeführt ist, dergestalt, dass er im zentralen Teil mindestens zwei rotationssymmetrische, je einen scheibenartigen Flansch (19; 20) und einen Hals (21; 22) aufweisende demontierbare, mit durch Anschlag (75) begrenzte Nut (23; 24) und Nocken (25; 26) versehene, koaxial ineinander steckbare Bauteile (17; 18) mit Schnapp- und Greifmechanismen für die gegenseitige Fixierung und Halterung der Gasentwicklungszellen (8) und der als Dreh-Potentiometer ausgeführten Widerstände (9) enthält, wobei mindestens eines der steckbaren Bauteile (17) mit einem profilierten Zapfen (15) ausgerüstet ist, der in eine entsprechende Oeffnung des Dreh-Potentiometers mit einem Schiebesitz eingreift.

4. Vorrichtung nach Anspruch 3, dadurch gekennzeichnet, dass unter den im zentralen Teil des Behälterbodens (11) untergebrachten rotationssymmetrischen Bauteilen (17; 18) ein erstes inneres, mit einer Klemmeinrichtung (12) mit nach innen gerichteten federnden Zungen zur Halterung der zylindrischen knopfförmigen Gasentwicklungszellen (8) und des drehbaren Einstellwiderstandes (9) versehenes stopfenförmiges Bauteil (18) mit einem scheibenartigen Flansch (20) mit peripherem, in eine Nut (77) mit Anschlag (78) des Behälterbodens (11) eingreifenden Nocken (76) und einem rohrartigen Hals (22) mit Aussengewinde vorhanden ist, welches in ein entsprechendes Innengewinde in einer zentralen Oeffnung des abgesetzten Behälterbodens (11) einschraubbar ist, und dass ein zweites äusseres ebenfalls stopfenförmiges mit Markierungspfeil (81) zu Skala versehenes Bauteil (17) vorhanden ist, das mittels eines rohrartigen, radial nachgiebigen Schnappverschlusses ins erste steckbar gestaltet ist, und dass ferner sowohl zwischen dem Behälterboden (11) über eine kreisförmige Vertiefung und dem ersten Bauteil (18) wie auch zwischen diesem ebenfalls über eine Vertiefung und dem zweiten Bauteil (17) je ein O-Ring (27; 16) als Dichtung angeordnet ist, dass ferner eine Nachfülleinrichtung (79) in Form einer faltenbalgartigen Patrone mit Innengewindeansatz vorgesehen ist.

5. Vorrichtung nach Anspruch 1, dadurch gekennzeichnet, dass der die elektrischen Elemente (8; 9; 10) enthaltende Behälterboden (11) geteilt ausgeführt ist, wobei ein äusseres, mit den benachbarten Bauelementen durch O-Ringe (64; 65) abgedichtetes, mit dem zylindrischen Behälterende verschraubbares, eine Skala (80) aufweisendes topfartiges Bauteil (28) mit einer über einen Einrastwulst (89) aufgeklemmten mit radial nach innen und axial nach aussen wirkenden, zentralsymmetrisch angeordneten Halte- und Federklammer (29) zur Halterung der Einzelzelle (8) sowie ein axial angeordneter Kontakt (30) mit radialer Kontaktzunge vorhanden ist, während ein inneres, in das äussere Bauteil (28) einpressbares, einen Markierpfeil (81) zur Skala aufweisendes rotationssymmetrisches Bauteil (31) einen kreisringförmigen Einstellwiderstand (9) und einen zentralen, mit diesem fest verbundenen Kontakt (66) trägt.

6. Vorrichtung nach Anspruch 1, dadurch gekennzeichnet, dass der zylindrische Behälter (1) dem graduierten Gefäss einer standardisierten Infusionsspritze entspricht und am bodenseitigen Ende einen nach innen vorspringenden Wulst (34) besitzt, dass ein aus mehreren Teilen bestehender Kolben (6) mit einem Kolbenkern (82) mit Gewinde (85) für Betätigungsstange zum Aufziehen des Mediums sowie mit radial stehenden fahnenförmigen Führungsrippen (46) vorgesehen ist, und dass das den Behälterboden (11) bildende, die elektrischen Elemente (8; 9; 10) enthaltende Bauteil aus einer aussen liegenden, mit Anschlagring (49), stirnseitiger Dichtungslippe (83), nach innen vorspringendem Anschlagwulst (84) und mehreren innen liegenden konischen Absetzungen (51) versehenen rotationssymmetrischen radial elastischen Hülse (48) und einem innen liegenden, an seinem offenen Ende mit einem radial/axial federnden inneren Verriegelungs/Einrast-Ring (54) sowie Entlüftungsöffnungen (44) zur Druckentlastung versehenen rotationssymmetrischen hohlen Press/Dichtungs-Stopfen (42) besteht, der in seiner äusseren Stirnseite ein Innengewinde (86) für Betätigungsstange zur Demontage trägt, und dass schliesslich die elektrischen Elemente (8; 9; 10) aus einer axial gelagerten zylindrischen Einzelzelle (8), einem kurzen, axial stehenden festen elektrischen Stabwiderstand (9) und aus als U/S-förmig gekrümmten Bändern gestalteten federnden Kontakten (10) bestehen.

7. Vorrichtung nach Anspruch 1, dadurch gekennzeichnet, dass der zylindrische Behälter (1) dem graduierten Gefäss einer in den wesentlichen Abmessungen standardisierten Infusionsspritze entspricht, am bodenseitigen Ende eine nach aussen vorkragende konische Absetzung (56) und einen nach innen vorspringenden Wulst (34) besitzt, dass ein Kolben (6) vorgesehen ist, und dass das den Behälterboden (11) bildende, die elektrischen Elemente (8; 9; 10) enthaltende Bauteil aus einer einzigen rotationssymmetrischen, im wesentlichen radial elastischen Kappe (59) als Abschlussdeckel mit einer innenliegenden federnden Dichtungslippe (60) und einer innenliegenden konischen Eindrehung (61), die in die konische Absetzung (56) des Behälters (1) eingreift, sowie mit radial nach innen und axial nach aussen wirkenden, zentralsymmetrisch angeordneten Halte- und Federklammern (29) zur Halterung der Einzelzelle (8) und mit axial stehenden Führungsrippen (62) für die bügelförmigen Kontakte (10) besteht, und dass schliesslich die elektrischen Elemente (8; 9; 10) aus einer axial gelagerten zylindrischen Einzelzelle (8), einem kurzen, axial stehenden festen elektrischen Stabwiderstand (9) und aus als doppel-S-förmig gekrümmte Bänder gestalteten federnden Kontakten (10) besteht.

8. Vorrichtung nach Anspruch 1, dadurch gekennzeichnet, dass der mit Dichtung (87) abgeschlossene zylindrische Behälter (1) mit einer aus einem U-förmigen Gehäuse (67), einem axialen Anschlagkontakt (68), einem radialen Kontakt (88) zwischen Gasentwicklungszelle (8) und Gehäuse (67), Klemmbacken (69), einer linearen Spule (70), Tastknöpfen (71) und einer Anzeige (72) bestehenden Wegmess-Einrichtung mechanisch fest über die Klemmbacken (69) und den Anschlagkontakt (68) und über die Kontakte (68; 88) ausserdem elektrisch verbunden ist, und dass der Kolben (6) in seinem Innern mit einem auf die Spule (70) einwirkenden Dauermagneten (73) bestückt ist.

## Claims

1. Device for selective controllable release of a fluid or a viscous mass or a suspension of solid particles in a fluid, consisiting of
a) a cylindrical container (1) with sliding fit for a cylindrical piston (6),
b) a container seal (2) wiht screw or plug connection (3),
c) said cylindrical piston (6) fitting into the container (1) with sliding fit, dividing said container into a pressurized gas chamber (4) and a mass chamber (5) having a circumferential seal (7),
d) a component containing the electrical elements such as electrochemical gas evolution cells (8), adjusting and loading resistors (9) and contacts (10), wherein
- said gas evolution cell (8) shows a tablet-shaped construction metallically closed from all sides, has a gas diffusion electrode, an aqueous electrolyte and a counterelectrode and can be activated by closing an external current circuit,
- said component containing said electrical element (8; 9; 10) is the container bottom (11) forming a monolithic structure or being connected to said container (1) via a releasable screw or plug connection, and said electrical elements (8; 9; 10) are held by a clamping device (12) and a funnel-shaped container seal (2) is provided at the mass-chamber end of said container (1).
characterized in that
(e) said circumferential seal has a radially prebiased sealing lip (7, 83).

2. Device in accordance with Claim 1, characterized by the fact that the piston (6) on the pressurized gas chamber side is provided with a protruding ring-shaped sealing lip (7) creating a space (74) for a grease film and in the central part a pot-like recess/depression (13) which reduces the pressurized gas space (4) to a minimum, and the container seal (2) is designed in the form of a funnel-shaped elastic corrugated sealing cover with a threaded nipple and that the electrical elements (8, 9, 10) designed in cornpact form as a double cell with electrical resistor are held by a clamping device (12) directly in the container bottom (11) provided with a marking arrow (81) for the scale, and that also in the container bottom (11) a scale-(80)-equipped and O-ring-(60)-sealed rotating plug-shaped component (17) displaying a pipe-like neck (21) is provided for adjusting the electrical resistance (9) designed as a rotary potentiometer which has a profiled pivot/peg (15) which engages a corresponding opening in the rotary potentiometer with a sliding fit.

3. Device in accordance with Claim 1, characterized by the fact that the piston (6) on the pressure chamber side is provided with a chamber (74) for a protruding annular sealing lip (7) creating a film of grease and in the central part with a pot-like recess/depression (13) reducing the pressurized gas chamber (4) to a minimum, and the container seal (2) is designed in the form of a funnel-shaped elastic corrugated sealing lid with a threaded nipple and that the electrical elements (8, 9, 10) designed as a double cell with electrical resistor in compact form is held by a clamping device (12) indirectly in the container bottom (11) provided with the scale (80), where the container bottom is designed to be multiply divided for the purpose of exchangeability and replaceability of the electrical elements (8, 9, 10) and refillability of the medium to be conveyed in such a way that it contains in the central part at least two rotationally symmetrical components (17, 18) which can be plugged into one another coaxially, each displaying a disk-like flange (19, 20) and a neck (21, 22), can be disassembled, and are provided with a groove (23, 24) limited by a stop (75) and cams (25, 26), with snapping and gripping mechanisms for mutual fixation and holding of the gas evolution cells (8) and which contains resistors (9) designed as a rotary potentiometer, where at least one of the pluggable components (17) is equipped with a profiled pivot/peg (15) which engages a corresponding opening of the rotary potentiometer with a sliding fit.

4. Device in accordance with Claim 3, characterized by the fact that among the rotationally symmetrical components (17, 18) installed in the central part of the container bottom (11) a first interior plug-shaped component (18) provided with a damping device (12) with inwardly directed resilient tongues to hold the cylindrical knob shaped gas evolution cells (8) and rotatable adjusting resistor (9) with a disk-like flange (20) with peripheral cam (76) engaging a groove (77) with stop (78) of the container bottom (11) and a pipe-like neck (22) with external threading is present, which can be screwed into a corresponding internal threading in a central opening in the offset container bottom (11), and that a second outer component (17) also plug-shaped with a marking arrow (81) for the scale is present which by means of a pipe-like radially flexible snapping closure can be made to plug into the first component and that also both between the container bottom (11) via a circular depression and the first component (18) and also between the container bottom via a depression and the second component (17) one O-ring each (27, 16) is arranged as a seal, that in addition a refilling device (79) is provided in the form of a folding bellows-like cartridge with internal threading attachment.

5. Device in accordance with Claim 1, characterized by the fact that the container bottom (11) containing electrical elements (8, 9, 10) is divided where an outer pot-like component (28) displaying a scale (80) capable of being screwed to the cylindrical container end, sealed against the neighboring structural elements by O-rings (64, 65) is present with radially inwardly and axially outwardly acting centrally-symmetrically arranged spring leaf retainers (29) clamped in via a snap-in bulge (89) is present to hold the single cell (8) and an axially arranged contact (30) with a radial contact tongue while an inner rotationally symmetrical component (31) capable of being pressed into the outer component (28) and displaying a marking arrow (81) for the scale carries a circular-ring-shaped adjusting resistor (9) and a central contact (66) firmly connected to it.

6. Device in accordance with Claim 1, characterized by the fact that the cylindrical container (1) corresponds to the graduated vessel of a standardized infusion syringe and on the bottom side end has an inwardly projecting bulge (34), that a piston (6) consisting of several parts with a piston core (82) with threading (85) for activation rods for pulling up the medium and with a radially positioned vane-shaped guide rib (46) is provided, and that the component forming the container bottom (11) containing the electrical elements (8, 9, 10) consists of an outer-lying rotationally symmetrically radially elastic sleeve (48) provided with a stop ring (49), inside sealing lip (83), inwardly projecting stop bulge (84) and several inwardly lying conical shoulders (51) and an inwardly-lying rotationally symmetrical hollow pressing-sealing plug (42) provided at its open end with a radially/axially resilient internal locking/snap-in ring (54) and air evacuation openings (44) for pressure relief, which carries in its outer end side an internal threading (86) for activation rods for disassembly, and that finally the electrical elements (8, 9, 10) consist of an axially mounted cylindrical single cell (8), a short axially positioned fixed electrical rod resistor (9) and of springing contacts formed of bands curved in a U-/S-shape.

7. Apparatus in accordance with Claim 1, characterized by the fact that the cylindrical container (1) corresponds to the graduated vessel of an infusion syringe standardized in its essential dimensions, has on the bottom side end an outwardly projecting conical shoulder (56) and an inwardly projecting bulge (34), that a piston (6) is provided and that the component forming the container bottom (11) which contains the electrical elements (8, 9, 10) consists of a single rotationally symmetrically essentially radially elastic cap (59) as a closing lid with an inner-lying resilient sealing lip (60) and an inner-lying conical recess (61) which engages the conical shoulder (56) of the container (1) and with radially inwardly and axially outwardly acting centrally symmetrically arranged spring leaf retainers (29) for holding the single cell (8) and with axially positioned guide ribs (62) for the strap-shaped contacts (10), and that finally the electrical elements (8, 9, 10) consist of an axially mounted cylindrical single cell (8), a short axially positioned fixed electrical rod resistor (9) and of resilient contacts (10) designed as double S-shaped curved bands.

8. Device in accordance with Claim 1, characterized by the fact that the cylindrical container (1) closed by the seal (87) is connected mechanically firmly with a path-measuring device consisting of a U-shaped housing (67), an axial stop contact (68), a radial contact (88) between gas evolution cell (8) and housing (67), clamp jaws (69), a linear coil (70), push buttons (71) and an indicator (72), via the damp jaws (69) and the stop contact (68), and also electrically connected via the contacts (68, 88) and that the piston (6) is equipped in its interior with a permanent magnet (73) which acts on the coil (70).

## Revendications

1. Dispositif permettant de distribuer un liquide ou une matière visqueuse ou une suspension de particules solides dans un liquide de manière ciblée et contrôlée, constitué de
a) un récipient cylindrique (1) présentant une assise coulissante pour un piston cylindrique (6),
b) une fermeture de récipient (2) avec un raccord à visser ou à enficher (3),
c) le piston cylindrique (6) qui s'ajuste dans le récipient (1) avec une assise coulissante et qui divise le récipient (1) en un espace de gaz sous pression (4) et en un espace de masse (5), étant muni d'une garniture d'étanchéité périphérique,
d) un composant contenant les éléments électriques comme des cellules électrochimiques (8) de dégagement de gaz, des résistances de réglage et de charge (9) et des contacts (10), étant précisé que
- la cellule (8) de dégagement de gaz présente une structure en forme de tablette, close de tous côtés par du métal, possède une électrode de diffusion du gaz, un électrolyte aqueux et une contre-électrode et peut s'activer par fermeture d'un circuit électrique extérieur,
- le composant contenant les éléments électriques (8;9;10) constitue le fond (11) du récipient, formant avec le récipient (1) une structure monolithique ou relié au récipient (1) au moyen d'une liaison à visser ou à enficher, détachable, et les éléments électriques (8;9;10) sont tenus au moyen d'un mécanisme d'accrochage (12) et une fermeture du récipient (2) en forme d'entonnoir est prévue à l'extrémité du récipient (1) située du côté de l'espace de masse,
caractérisé par le fait que
e) la garniture périphérique présente une lèvre d'étanchéité (7,83) précontrainte radialement.

2. Dispositif selon la revendication 1, caractérisé par le fait que le piston (6) comporte, du côté de l'espace de gaz sous pression, une lèvre d'étanchéité annulaire (7) qui saille et crée un espace (74) pour un film de graisse et, dans la partie centrale, un évidement/creux (13) en forme de boisseau qui réduit au minimum l'espace du gaz sous pression (4) et la fermeture du récipient (2) est réalisée sous forme d'un couvercle de fermeture ondulé, élastique, en forme d'entonnoir, avec un raccord fileté et que les éléments électriques (8;9;10), réalisés de façon compacte sous forme de double cellule avec résistance électrique, sont directement tenus, au moyen d'un mécanisme d'accrochage (12), dans le fond (11) du récipient, muni d'une flèche de marquage (81) par rapport à une graduation, et qu'est prévu en outre, dans le fond du récipient (11), un composant rotatif en forme de bouchon (17), équipé d'une graduation (80) et présentant un col tubulaire (21) rendu étanche par un joint torique (16), pour régler la résistance électrique (9) qui est réalisée sous forme d'un potentiomètre rotatif et possède un téton profilé (15) qui vient en prise, par une assise coulissante, dans une ouverture correspondante du potentiomètre rotatif.

3. Dispositif selon la revendication 1, caractérisé par le fait que le piston (6) comporte, du côté de l'espace de gaz sous pression, une lèvre d'étanchéité annulaire (7) qui saille et crée un espace (74) pour un film de graisse et, dans la partie centrale, un évidement/creux (13) en forme de boisseau qui réduit au minimum l'espace du gaz sous pression (4) et la fermeture du récipient (2) est réalisée sous forme d'un couvercle de fermeture ondulé, élastique, en forme d'entonnoir, avec un raccord fileté et que les éléments électriques (8; 9; 10), réalisés de façon compacte sous forme de double cellule avec résistance électrique, sont indirectement tenus, au moyen d'un mécanisme d'accrochage (12), dans le fond du récipient (11) muni d'une graduation (80), ce dernier étant, aux fins de possibilité d'échange et de possibilité de renouvellement des éléments électriques (8, 9; 10) et de possibilité de refaire le plein du fluide à transporter, réalisé subdivisé plusieurs fois, de façon à contenir, dans la partie centrale, au moins deux composants (17;18) présentant une symétrie de rotation, présentant chacun un rebord en forme de disque (19; 20) et un col (21; 22), démontables, munis d'une rainure (23; 24) limitée par une butée (75) et d'un saillant (25; 26), coaxialement enfichables l'un dans l'autre, avec des mécanismes de fixation par déformation élastique et de saisie pour la fixation et le maintien mutuels des cellules de développement de gaz (8) et des résistances (9) réalisées sous forme de potentiomètres rotatifs, au moins l'un des composants enfichables (17) étant muni d'un téton profilé (15) qui vient en prise, par une assise coulissante, dans une ouverture correspondante du potentiomètre rotatif.

4. Dispositif selon la revendication 3, caractérisé par le fait que sous les composants (17; 18) présentant une symétrie de rotation, logés dans la partie centrale du fond du récipient (11), il y a un premier composant intérieur en forme de bouchon (18) qui est muni d'un mécanisme d'accrochage (12), présentant des languettes élastiques orientées vers l'intérieur, pour tenir les cellules cylindriques, en forme de bouton, de développement de gaz (8) et la résistance rotative de réglage (9), avec un rebord (20) en forme de disque, avec un saillant périphérique (76) qui vient en prise dans une rainure (77) avec butée (78) du fond du récipient (11) et avec un col tubulaire (22) à filetage extérieur, qui peut se visser dans un filetage intérieur correspondant prévu dans une ouverture centrale du fond du récipient (11), qui présente un décrochement, et qu'il y a un second composant extérieur (17), également en forme de bouchon, qui est muni d'une flèche de marquage (81) par rapport à une graduation et qui est réalisé de façon à pouvoir s'enficher dans le premier composant au moyen d'une fermeture tubulaire à déformation élastique qui cède radialement, et qu'en outre, aussi bien entre le fond du récipient (11), au moyen d'une rainure circulaire, et le premier composant (18) qu'entre celui-ci, également au moyen d'une rainure, et le second composant (17), est chaque fois disposé comme garniture d'étanchéité un joint torique (27;16), qu'est prévu en outre un mécanisme permettant de refaire le plein (79), sous forme d'une cartouche en accordéon avec embout fileté intérieurement.

5. Dispositif selon la revendication 1, caractérisé par le fait que le fond (11) du récipient qui contient les éléments électriques (8; 9; 10) est réalisé divisé, étant précisé qu'il existe un composant extérieur (28) en forme de boisseau, rendu étanche à l'égard des composants voisins par des joints toriques (64; 65), pouvant se visser dans l'extrémité cylindrique du récipient, présentant une graduation (80), avec une attache de maintien élastique (29), disposée avec symétrie centrale et agissant radialement vers l'intérieur et axialement vers l'extérieur, pour tenir la cellule individuelle (8) ainsi qu'un contact (30), disposé axialement, avec des languettes de contact radiales, tandis qu'un composant intérieur (31), qui présente une symétrie de rotation, peut se monter à ajustement serré dans le composant extérieur (28) et présente une flèche de marquage (80) par rapport à la graduation, porte une résistance réglable (31) en forme d'anneau circulaire et un contact central (66) qui lui est solidarisé.

6. Dispositif selon la revendication 1, caractérisé par le fait que le récipient cylindrique (1) correspond au récipient gradué d'une seringue standardisée pour perfusion et possède, à son extrémité située du côté du fond, un bourrelet (34) qui déborde vers l'intérieur, qu'il est prévu un piston (6), constitué de plusieurs pièces, avec un noyau de piston (82) à filetage (85) pour la tige de manoeuvre prévue pour faire monter le fluide, ainsi qu'avec des nervures de guidage (46) en forme de palette placées radialement, et que le composant qui forme le fond (11) du récipient et contient les éléments électriques (8; 9; 10) est constitué d'une gaine (48), en symétrie de rotation, radialement élastique, située à l'extérieur, présentant une bague de butée (49), une lèvre d'étanchéité frontale (83), un bourrelet de butée (84) débordant vers l'intérieur et plusieurs décrochements coniques (51) situés à l'intérieur, ainsi que d'un bouchon creux de serrage/d'étanchéité (42), qui est situé à l'intérieur, qui présente une symétrie de rotation, qui est muni, à son extrémité ouverte, d'une bague intérieure de verrouillage/de crantage (54), élastique dans le sens radial et axial, ainsi que d'ouvertures de purge (44) pour la décharge de pression, qui porte, sur sa face frontale extérieure, un filetage intérieur (86) pour une tige de manoeuvre pour le démontage, et par le fait qu'enfin les éléments électriques (8; 9; 10) sont constitués d'une cellule individuelle cylindrique disposée axialement, d'une courte résistance électrique droite, rigide, (9), disposée axialement, et de contacts élastiques réalisés sous forme de rubans incurvés en forme de U/S.

7. Dispositif selon la revendication 1, caractérisé par le fait que le récipient cylindrique (1) correspond au récipient gradué d'une seringue standardisée pour perfusion en ce qui concerne les dimensions essentielles, possède, à son extrémité située du côté du fond, un décrochement conique (56) qui part en saillie vers l'extérieur et un bourrelet (34) qui déborde vers l'intérieur, qu'il est prévu un piston (6), et que le composant qui forme le fond (11) du récipient et contient les éléments électriques (8; 9; 10) est constitué d'un unique capuchon (59), présentant une symétrie de rotation, essentiellement élastique dans le sens radial, comme couvercle d'obturation, avec une lèvre d'étanchéité élastique (60) située à l'intérieur et une saignée conique (31) qui est située à l'intérieur et vient en prise dans le décrochement conique (56) du récipient (1), ainsi qu'avec des attaches de maintien élastique (29), disposées symétriquement par rapport au centre, agissant radialement vers l'intérieur et axialement vers l'extérieur, pour tenir la cellule individuelle (8) et avec des nervure de guidage (62), placées axialement, pour les contacts en forme d'étrier (10), et qu'enfin les éléments électriques (8; 9; 10) sont constitués d'une cellule individuelle cylindrique disposée axialement, d'une courte résistance électrique droite, rigide, (9), disposée axialement, et de contacts élastiques réalisés sous forme de rubans incurvés en forme de double S.

8. Dispositif selon la revendication 1, caractérisé par le fait que le récipient cylindrique (1) obturé avec étanchéité (87) est mécaniquement solidarisé, par l'intermédiaire des mors de bridage (69) et des contacts de butée (68), avec un dispositif de mesure de la course constitué d'un boîtier (67) en forme de U, d'un contact axial de butée (68), d'un contact radial (88) entre la cellule (8) de dégagement de gaz et le boîtier (67), de mors de bridage (69), d'une bobine linéaire (70), de boutons de manoeuvre (71) et d'un visuel (72) et qu'il est en outre relié électriquement par l'intermédiaire des contacts (68; 88), et que le piston (6) est garni intérieurement d'un aimant permanent (73) agissant sur la bobine (70).
